(19) Europäisches Patentamt
European Patent Office
Office européen des brevets

(11) EP 0 624 572 B1

(12) **EUROPÄISCHE PATENTSCHRIFT**

(45) Veröffentlichungstag und Bekanntmachung des Hinweises auf die Patenterteilung:
**04.09.1996 Patentblatt 1996/36**

(51) Int. Cl.$^6$: **C07C 323/52**, A61K 7/09

(21) Anmeldenummer: **94104917.3**

(22) Anmeldetag: **29.03.1994**

(54) **Mittel zur dauerhaften Haarverformung sowie neue haarkeratinreduzierende 2-Hydroxy-3-mercaptopropionsäureester**

Agent for the permanent reshaping of hair and new hair keratin reducing 2-hydroxy-3-mercatopropionic esters

Compositions pour la déformation permanente des cheveux ainsi que des nouveaux esters 2-hydroxy-3-mercaptopropionique réductrices de la kératine des cheveux

(84) Benannte Vertragsstaaten:
**DE ES FR GB IT**

(30) Priorität: **12.05.1993 DE 4315840**

(43) Veröffentlichungstag der Anmeldung:
**17.11.1994 Patentblatt 1994/46**

(73) Patentinhaber: **Wella Aktiengesellschaft**
**D-64274 Darmstadt (DE)**

(72) Erfinder:
- **Braun, Hans-Jürgen, Dr.**
  **CH-3182 Ueberstorf (CH)**
- **Clausen, Thomas, Dr.**
  **D-64665 Alsbach (DE)**
- **Maresch, Gerhard**
  **D-64295 Darmstadt (DE)**

(56) Entgegenhaltungen:
**EP-A- 0 235 783**
**WO-A-93/01791**
**DE-A- 2 658 424**
**DE-A- 3 920 984**
**DE-A- 4 003 234**

- **PATENT ABSTRACTS OF JAPAN, vol. 3, no. 72 (C-049), 21. Juni 1979, & JP-A-54 044 041**
- **JOURNAL OF MEDICINAL CHEMISTRY, Bd. 29, Nr. 9, September 1986, Washington, DC, US; Seiten 1610 - 1615; E. TEODORI et al: "Resolution, absolute configuration, and cholinergenic enantioselectivity of (+)- and (-)-cis-2-methyl-5-[(dimethylamino)methyl]-1,3-oxathiolane methiodide"**
- **JOURNAL OF THE AMERICAN CHEMICAL SOCIETY, Bd. 52, Nr. 3, März 1930, Washington, DC, US, Seiten 1105 - 1108, F. KOELSCH: "A sulphur analogue of glyceric acid. Beta-thioglyceric acid"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 624 572 B1

Printed by Rank Xerox (UK) Business Services
2.13.4/3.4

## Beschreibung

Die vorliegende Erfindung betrifft ein Mittel zur dauerhaften Haarverformung sowie neue haarkeratinreduzierende 2-Hydroxy-3-mercaptopropionsäureester.

Für eine schonende dauerhafte Verformung von geschädigtem, insbesondere gebleichtem oder gefärbtem Haar werden bevorzugt schwach sauer bis neutral eingestellte Verformungsmittel verwendet. Hierbei haben sich im Verlauf der letzten 30 Jahre die Thioglykolsäureester als für diesen Zweck am besten geeignete Reduktionsmittel erwiesen.

Dem Vorteil einer haarschonenderen Verformungsbehandlung durch schwach saure bis neutrale Verformungsmittel stehen jedoch eine Reihe von Nachteilen gegenüber. So besitzen diese Mittel eine im Vergleich zu mildalkalischen Verformungsmitteln auf Thioglykolatbasis geringere Wellwirksamkeit. Aus diesem Grunde ist für die Erzielung einer ausreichenden Umformung die Zufuhr von Wärme, eine Verlängerung der Einwirkungszeiten auf 20 bis 60 Minuten sowie die Verwendung von vergleichsweise dünnen Wicklern erforderlich. Eine Verwendung dieser Verformungsmittel für normales, nicht geschädigtes Naturhaar erscheint aufgrund der auch bei Wärmezufuhr erforderlichen langen Einwirkungszeiten von über 30 Minuten nicht sinnvoll, so daß die Anwendung von schwach sauer bis neutral eingestellten Verformungsmitteln bisher in der Regel auf vorgeschädigtes, leicht verformbares Haar beschränkt blieb.

Ein weiterer erheblicher Nachteil von sauren Haarverformungsmitteln ist die schlechte Augen- und Hautverträglichkeit sowie die sensibilisierende Wirkung der Thioglykolsäureester.

In der DE-OS 39 20 984 und der DE-OS 40 03 234 wurde daher vorgeschlagen, die sensibilisierende Wirkung von sauren Haarverformungsmitteln auf Mercaptocarbonsäureester-Basis durch eine Kombination des Thioglykolsäureesters mit 2-Mercaptopropionsäuremonoglycerinestern oder/und 3-Mercaptopropionsäuremonoglycerinestern beziehungsweise durch die alleinige Verwendung dieser Mercaptopropionsäureester herabzusetzen.

Es hat sich jedoch gezeigt, daß hierdurch die sensibilisierende Wirkung von sauren Haarverformungsmitteln nicht entscheidend verringert werden kann.

Der Erfindung liegt daher die Aufgabe zugrunde, ein Mittel zur dauerhaften Haarverformung zur Verfügung zu stellen, das sowohl im sauren als auch schwach alkalischen Bereich (pH-Wert = 4,5 bis 9,0) eine schonende und gleichmäßige Umformung ermöglicht und darüber hinaus kein oder aber nur ein geringes Allergiepotential aufweist.

Überraschenderweise wurde nunmehr gefunden, daß mit Mitteln zur dauerhaften Verformung von Haaren auf der Basis eines 2-Hydroxy-3-mercaptopropionsäureesters der Formel (I)

$$HS\text{-}CH_2\text{-}CH(OH)\text{-}COOR \qquad (I)$$

mit R = einer Alkylgruppe, vorzugsweise einer $C_1$- bis $C_4$-Alkylgruppe; einer Hydroxyalkylgruppe, vorzugsweise einer $C_2$- bis $C_4$-Hydroxyalkylgruppe, oder einer Dihydroxyalkylgruppe, vorzugsweise einer $C_3$- oder $C_4$-Dihydroxyalkylgruppe;

eine gleichmäßige Umformung sowohl von geschädigtem als auch ungeschädigtem Haar möglich ist, ohne daß hierbei die bei den bisher für die Haarverformung verwendeten haarkeratinreduzierenden Esterverbindungen häufig beobachteten allergischen Hautreaktionen auftreten.

Gegenstand der vorliegenden Erfindung ist daher ein Mittel zur dauerhaften Verformung von Haaren auf der Basis eines keratinreduzierenden Wirkstoffes, welches als keratinreduzierenden Wirkstoff einen 2-Hydroxy-3-mercaptopropionsäureester der Formel (I) enthält.

Als 2-Hydroxy-3-mercaptopropionsäureester der Formel (I) können insbesondere der 2-Hydroxy-3-mercaptopropionsäureethylester, der 2-Hydroxy-3-mercaptopropionsäure-(2'-hydroxyethyl)ester und der 2-Hydroxy-3-mercaptopropionsäure-(2',3'-dihydroxypropyl)ester verwendet werden.

Zwar ist es möglich den 2-Hydroxy-3-mercaptopropionsäureester der Formel (I) gemeinsam mit anderen keratinreduzierenden Wirkstoffen - wie zum Beispiel Thioglykolsäure, Thiomilchsäure, 3-Hydroxy-2-mercaptopropionsäure, Cysteamin und Cysteaminderivaten oder Cystein und Cysteinderivaten - zu verwenden, jedoch ist die Verwendung des 2-Hydroxy-3-mercaptopropionsäureesters als alleinigem keratinreduzierenden Wirkstoff (das heißt ohne Zusatz anderer keratinreduzierender Wirkstoffe) besonders bevorzugt.

Der 2-Hydroxy-3-mercaptopropionsäureester der Formel (I) wird in dem gebrauchsfertigen erfindungsgemäßen Mittel zur dauerhaften Haarverformung in einer Menge von 4 bis 30 Gewichtsprozent, vorzugsweise in einer Menge von 8 bis 28 Gewichtsprozent, eingesetzt.

Das gebrauchsfertige erfindungsgemäße Verformungsmittel besitzt einen pH-Wert von 4,5 bis 9,0, vorzugsweise von 6,0 bis 7,5.

Das Verformungsmittel kann sowohl in Form einer wäßrigen Lösung oder einer Emulsion als auch in verdickter Form auf wäßriger Basis, insbesondere als Gel, Creme oder Paste vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den

Klassen der anionischen, kationischen amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle; Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Da Carbonsäureester in wäßrigem Medium nur für begrenzte Zeit haltbar sind und, insbesondere im alkalischen Bereich, leicht hydrolysierten, wird der 2-Hydroxy-3-mercaptopropionsäureester der Formel (I) vorzugsweise in wasserfreier Form abgepackt und das Verformungsmittel erst unmittelbar vor Gebrauch durch Vermischen der den 2-Hydroxy3-mercaptopropionsäureester enthaltenden Komponente mit einer oder mehreren weiteren Komponenten hergestellt.

Je nach Konfektionierung kann das erfindungsgemäße Mittel hierbei in Form eines Zwei- oder Dreikomponenten-Präparates vorliegen.

So ist das erfindungsgemäße Mittel zum Beispiel durch Vermischen zweier Komponenten erhältlich, von denen die erste Komponente das oder die Alkalisierungsmittel, beispielsweise ein Alkalicarbonat, Ammoniumcarbonat, Alkalihydrogencarbonat oder Ammoniumhydrogencarbonat, sowie die vorstehend genannten kosmetischen Zusatzstoffe und Wasser enthält, während die zweite, wasserfreie Komponente den 2-Hydroxy-3-mercaptopropionsäureester enthält.

Ebenfalls ist es möglich, das erfindungsgemäße Mittel in Form eines Dreikomponenten-Präparates abzupacken, wobei eine Komponente einen Teil der vorstehend genannten kosmetischen Zusatzstoffe sowie Wasser, eine zweite, wasserfreie Komponente den 2-Hydroxy-3-mercaptopropionsäureester und die dritte Komponente Parfümöle, Lösungsvermittler und Pflegestoffe in wäßriger Lösung oder wasserfreier Form enthält.

Bei allen Ausführungsformen des erfindungsgemäßen Mittels können die vorstehend genannten kosmetischen Zusatzstoffe sowohl in der wäßrigen als auch in der/den nichtwäßrigen Komponente(n) enthalten sein.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte, gleichmäßige Umformung vom Haaransatz bis zur Haarspitze.

Die dauerhafte Verformung der Haare wird in der üblichen Weise durchgeführt, indem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet.

Hierbei kann das Haar zunächst mit einem Shampoo gewaschen und danach mit Wasser gespült werden. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die in den erfindungsgemäßen Mitteln zur Verformung der Haare enthaltenden 2-Hydroxy-3-mercaptocarbonsäureester der Formel (I) können in üblicher Weise durch Veresterung der 2-Hydroxy-3-mercaptopropionsäure in Gegenwart eines sauren Katalysators, beispielsweise Schwefelsäure oder Benzolsulfonsäure, mit einer geeigneten Monohydroxy-, Dihydroxy-oder Trihydroxyverbindung-insbesondere Ethanol, Ethylenglykol oder Glycerin-,vorzugsweise unter Wärmeeinwirkung, erhalten werden.

Die vorgenannten 2-Hydroxy-3-mercaptopropionsäureester der Formel (I) besitzen eine hohe Wellwirksamkeit bei sauren bis leicht alkalischen pH-Werten, sind nahezu geruchsneutral und leicht wasserlöslich und weisen eine hervorragende physiologische Verträglichkeit sowie eine gute Stabilität in Wasser auf.

Die Verbindungen der Formel (I) sind zum Teil aus der Literatur noch nicht bekannt.

Ein weiterer Gegenstand der vorliegenden Anmeldung sind daher die neuen 2-Hydroxy-3-mercaptopropionsäureester der Formel (II)

$$HS\text{-}CH_2\text{-}CH(OH)\text{-}COOR^1 \qquad (II),$$

mit $R^1$ = einer Alkylgruppe mit mindestens 3 C-Atomen, vorzugsweise einer $C_3$- oder $C_4$-Alkylgruppe ; einer Hydroxyalkylgruppe, vorzugsweise einer $C_2$- bis $C_4$-Hydroxyalkylgruppe; oder einer Dihydroxyalkylgruppe, vorzugsweise einer $C_3$- oder $C_4$-Dihydroxyalkylgruppe,

von denen der 2-Hydroxy-3-mercaptopropionsäure-(2'-hydroxyethyl)ester und der 2-Hydroxy-3-mercaptopropionsäure-(2',3'-dihydroxypropyl)ester besonders bevorzugt sind.

Die nachfolgenden Beispiele sollen den Gegenstand der Erfindung erläutern, ohne ihn hierauf zu beschränken.

**Beispiele für Dauerverformungsmittel**

**Beispiel 1: Einkomponenten-Dauerverformungsmittel**

| | |
|---|---|
| 8,0 g | 2-Hydroxy-3-mercaptopropionsäureethylester |
| 10,0 g | Ethanol |
| 82,0 g | Wasser |
| 100,0 g | |

Falls erforderlich wird der pH-Wert dieses Haarverformungsmittels vor Gebrauch mit einer physiologisch verträglichen schwachen Säure oder Base auf pH = 6,0 eingestellt.

Leicht vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 15 Minuten lang unter einer Trockenhaube bei einer Temperatur von 40 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, natürliche wirkende Umformung der Haare vom Haaransatz bis zu den Haarspitzen erhalten.

**Beispiel 2: Zweikomponenten-Dauerverformungsmittel**

Komponente 1:

| | |
|---|---|
| 0,3 g | Octylphenol, mit 20 Mol Ethylenoxid oxethyliert |
| 0,3 g | Parfümöl |
| 99,4 g | Wasser |
| 100,0 g | |

Komponente 2:

| | |
|---|---|
| 60,0 g | 2-Hydroxy-3-mercaptopropionsäure-(2'-hydroxyethyl)ester |
| 40,0 g | Isopropanol |
| 100,0 g | |

Vor dem Gebrauch werden 60 Gramm der Komponente 1 und 30 Gramm der Komponente 2 zu einem Haarverformungsmittel mit einem pH-Wert von 6,6 vermischt.

Leicht verformbares Haar wird mit einem Shampoo gewaschen und mit Wasser gründlich gespült. Anschließend wird das frottierte Haar auf Wickler mit einem Durchmesser von 10 Millimetern gewickelt und mit dem Haarverformungsmittel gleichmäßig befeuchtet. Nach einer Einwirkungszeit von 20 Minuten wird das Haar mit Wasser gespült und mit 100 Gramm einer 2-prozentigen Hydrogenperoxidlösung oxidativ nachbehandelt. Nach Entfernung der Wickler werden die Haare erneut mit Wasser gespült und danach getrocknet.

**Beispiel 3: Zweikomponenten-Dauerverformungsmittel**

Komponente 1:

| | |
|---|---|
| 2,0 g | Dipropylenglykolmonomethylether |
| 1,0 g | Ammoniak (25-prozentige wäßrige Lösung) |
| 0,3 g | Kokosfettalkohol, mit 10 Mol Ethylenoxid oxethyliert |
| 0,3 g | Parfümöl |
| 0,2 g | Diammoniumdithiodiglykolat |
| 96,2 g | Wasser, vollentsalzt |
| 100,0 g | |

Komponente 1:

| | |
|---|---|
| 60,0 g | 2-Hydroxy-3-mercaptopropionsäure-(2',3'-dihydroxypropyl)ester |
| 30,0 g | Glycerin |
| 10,0 g | N-Acetylcysteamin |
| 100,0 g | |

80 Gramm der Komponente 1 werden mit 40 Gramm der Komponente 2 zu einem gebrauchsfertigen Haarverformungsmittel vom pH 6,5 vermischt.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 20 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Das so umgeformte Haar besitzt eine über die gesamte Haarlänge gleichmäßige Krause, die mit der durch Behandlung mit mildalkalischen Dauerverformungsmitteln erzielten Krause vergleichbar ist.

**Herstellungsbeispiele für 2-Hydroxy-3-mercaptopropionsäureester**

**Beispiel 4: 2-Hydroxy-3-mercaptopropionsäure-(2'-hydroxyethyl)ester**

$$\underset{(122,14)}{HS-CH_2-CH(OH)-COOH} + \underset{(62,07)}{HO-CH_2-CH_2-OH} \xrightarrow[\text{Toluol}]{\text{Benzolsulfonsäure}}$$

$$\underset{(166,19)}{HS-CH_2-CH(OH)-COO(C_2H_4OH)} + \underset{(18,02)}{H_2O}$$

48,9 g (0,4 mol) 2-Hydroxy-3-mercaptopropionsäure werden gemeinsam mit 99,3 g (1,6 mol) Ethylenglykol und 0,43 g (2,7 mmol) Benzolsulfonsäure in 40 ml Toluol am Wasserabscheider 5 Stunden lang unter Rückfluß erwärmt. Anschließend wird die Reaktionsmischung in 200 ml Wasser gegossen, die organische Phase abgetrennt und verworfen. Sodann wird die wäßrige Phase neutralisiert und mit Diethylether extrahiert. Die Etherphase wird über Magnesiumsulfat getrocknet und sodann im Vakuum eingeengt. Durch Vakuumdestillation des zurückbleibenden Öles wird ein farbloses Öl mit einem Siedepunkt von 132 bis 134 Grad Celsius bei 0,04 mbar erhalten. Die Ausbeute beträgt 31,4 g (47 % der Theorie).

| CHS-Analyse: | | | |
|---|---|---|---|
| ($C_5H_{10}O_4S$) | % C | % H | % S |
| berechnet: | 36,14 | 6,07 | 19,29 |
| gefunden: | 36,25 | 6,17 | 19,27 |

$^1$H-NMR (in $CDCl_3$): = 1,73 (2 Dubletts, SH); 2,9 Multiplett); 3,9 (Multiplett), 4,4 (Multiplett); 4,5 (Multiplett)

**Beispiel 5: 2-Hydroxy-3-mercaptopropionsäure-(2',3'-dihydroxypropyl)ester**

$$HS\text{-}CH_2\text{-}CH(OH)\text{-}COOH + HO\text{-}CH_2\text{-}CH(OH)\text{-}CH_2OH \xrightarrow[\text{Toluol}]{\text{Benzolsulfon-säure}}$$

$$(122,14) \qquad (92,10)$$

$$HS\text{-}CH_2\text{-}CH(OH)\text{-}COO\text{-}CH_2\text{-}CH(OH)\text{-}CH_2OH + H_2O$$

$$(196,22) \qquad (18,02)$$

48,9 g (0,4 mol) 2-Hydroxy-3-mercaptopropionsäure werden gemeinsam mit 147,4 g (1,6 mol) Glycerin und 0,43 g (2,7 mmol) Benzolsulfonsäure in 40 ml Toluol am Wasserabscheider 5 Stunden lang unter Rückfluß erwärmt. Anschließend wird die Reaktionsmischung mit 0,5 ml 2-normaler Natronlauge neutralisiert und über Magnesiumsulfat getrocknet. Nachdem das Toluol abdestilliert worden ist, wird ein Öl erhalten, das noch etwa 25 Prozent freies Glycerin enthält und ohne weitere Reinigung in Dauerverformungsmitteln eingesetzt werden kann.

MS (FB +) m/e: 179, 161, 105

**Beispiel 6: 2-Hydroxy-3-mercoptopropionsäureethylester**

$$HS\text{-}CH_2\text{-}CH(OH)\text{-}COOH + C_2H_5OH \xrightarrow{H_2SO_4}$$

$$(122,14) \qquad (46,07)$$

$$HS\text{-}CH_2\text{-}CH(OH)\text{-}COOC_2H_5 + H_2O$$

$$(150,19) \qquad (18,02)$$

30,5 g (0,25 mol) 2-Hydroxy-3-mercaptopropionsäure werden in 300 ml (≙238,1 g = 5,2 mol) Ethanol unter Zusatz von 6,1 g konzentrierter Schwefelsäure in einer Stickstoffatmosphere bei Raumtemperatur gerührt. Anschließend wird ein Teil des Ethanols im Vakuum abdestilliert, der Rückstand in 400 ml Wasser aufgenommen und die Wasserphase mit Diethylether extrahiert. Anschließend wird die Etherphase abgetrennt, über Magnesiumsulfat getrocknet und sodann im Vakuum eingeengt.

Durch Vakuumdestillation des erhaltenen Öles wird ein farbloses Öl mit einem Siedepunkt von 56 bis 57 Grad Celsius bei 0,5 mbar (Lit.[1]: 113 bis 115 Grad Celsius bei 19 Torr) erhalten.
Ausbeute: 27,8 g (74 % der Theorie).

Alle in der vorliegenden Anmeldung enthaltenen Prozentangaben beziehen sich auf Gewichtsprozente, sofern nichts anderes angegeben ist.

**Patentansprüche**

1. Mittel zur dauerhaften Verformung von Haaren auf der Basis eines keratinreduzierenden Wirkstoffes, dadurch gekennzeichnet, daß es als keratinreduzierenden Wirkstoff einen 2-Hydroxy-3-mercaptopropionsäureester der Formel (I)

$$HS\text{-}CH_2\text{-}CH(OH)\text{-}COOR \qquad (I)$$

mit R = einer Alkyl-, einer Hydroxyalkyl- oder einer Dihydroxyalkylgruppe,

enthält.

2. Mittel nach Anspruch 1, dadurch gekennzeichnet, daß die Restgruppe R in Formel (I) eine $C_1$- bis $C_4$-Alkylgruppe, eine $C_2$- bis $C_4$-Hydroxyalkylgruppe oder eine $C_3$- oder $C_4$-Dihydroxyalkylgruppe ist.

3. Mittel nach Anspruch 1 oder 2, dadurch gekennzeichnet, daß es als alleinigen keratinreduzierenden Wirkstoff einen 2-Hydroxy-3-mercaptopropionsäureester der Formel (I) enthält.

4. Mittel nach einem der Ansprüche 1 bis 3, dadurch gekennzeichnet, daß der 2-Hydroxy-3-mercaptopropionsäureester der Formel (I) der 2-Hydroxy-3-mercaptopropionsäureethylester, der 2-Hydroxy-3-mercaptopropionsäure-(2'-hydroxyethyl)ester oder der 2-Hydroxy-3-mercaptopropionsäure-(2',3'-dihydroxypropyl)ester ist.

5. Mittel nach einem der Ansprüche 1 bis 4, dadurch gekennzeichnet, daß der 2-Hydroxy-3-mercaptopropionsäureester der Formel (I) in dem gebrauchsfertigen Mittel in einer Menge von 4 bis 30 Gewichtsprozent enthalten ist.

6. Mittel nach einem der Ansprüche 1 bis 5, dadurch gekennzeichnet, daß der pH-Wert des gebrauchsfertigen Mittels 4,5 bis 9,0 beträgt.

7. Mittel nach einem der Ansprüche 1 bis 6, dadurch gekennzeichnet, daß es vor der Anwendung durch Vermischen von zwei oder drei Komponenten, wobei die den 2-Hydroxy-3-mercaptopropionsäureester der Formel (I) enthaltende Komponente wasserfrei ist, hergestellt wird.

8. 2-Hydroxy-3-mercaptopropionsäureester der Formel (II)

$$HS\text{-}CH_2\text{-}CH(OH)\text{-}COOR^1 \qquad (II)$$

mit $R^1$ = einer Alkylgruppe mit mindestens 3 C-Atomen, einer Hydroxyalkylgruppe oder einer Dihydroxyalkylgruppe.

9. Verbindung gemäß Anspruch 8, dadurch gekennzeichnet, daß die Restgruppe $R^1$ in Formel (II) eine $C_3$- oder $C_4$-Alkylgruppe, eine $C_2$- bis $C_4$-Hydroxyalkylgruppe oder eine $C_3$- oder $C_4$-Dihydroxyalkylgruppe ist.

10. 2-Hydroxy-3-mercaptopropionsäure-(2'-hydroxyethyl)ester.

11. 2-Hydroxy-3-mercaptopropionsäure-(2',3'-dihydroxypropyl)ester.

**Claims**

1. Agent for the permanent shaping of hair based on a keratin-reducing active substance, characterised in that it contains as keratin-reducing active substance a 2-hydroxy-3-mercaptopropionic acid ester of formula (I)

[1](F. Koelsch in Journal of the American Chemical Society 1930 (52); Seite 1106)

$$HS\text{-}CH_2\text{-}CH(OH)\text{-}COOR \quad (I)$$

wherein R is an alkyl group, a hydroxyalkyl group or a dihydroxyalkyl group.

2. Agent according to Claim 1, characterised in that the residue R in formula (I) is a $C_1$- to $C_4$-alkyl group, a $C_2$- to $C_4$-hydroxyalkyl group or a $C_3$- or $C_4$-dihydroxyalkyl group.

3. Agent according to Claim 1 or 2, characterised in that it contains as the sole keratin-reducing active substance a 2-hydroxy-3-mercaptopropionic acid ester of formula (I).

4. Agent according to any one of Claims 1 to 3, characterised in that the 2-hydroxy-3-mercaptopropionic acid ester of formula (I) is 2-hydroxy-3-mercaptopropionic acid ethyl ester, 2-hydroxy-3-mercaptopropionic acid (2'-hydroxyethyl) ester or 2-hydroxy-3-mercaptopropionic acid (2',3'-dihydroxypropyl) ester.

5. Agent according to any one of Claims 1 to 4, characterised in that the amount of 2-hydroxy-3-mercaptopropionic acid ester of formula (I) present in the ready-for-use agent is from 4 to 30 % by weight.

6. Agent according to any one of Claims 1 to 5, characterised in that the pH value of the ready-for-use agent is from 4.5 to 9.0.

7. Agent according to any one of Claims 1 to 6, characterised in that it is prepared prior to use by mixing two or three components, the component containing the 2-hydroxy-3-mercaptopropionic acid ester of formula (I) being anhydrous.

8. 2-hydroxy-3-mercaptopropionic acid ester of formula (II)

$$HS\text{-}CH_2\text{-}CH(OH)\text{-}COOR^1 \quad (II)$$

wherein $R^1$ is an alkyl group having at least three carbon atoms, a hydroxyalkyl group or a dihydroxyalkyl group.

9. Compound according to Claim 8, characterised in that the residue $R^1$ in formula (II) is a $C_3$- or $C_4$-alkyl group, a $C_2$- to $C_4$-hydroxyalkyl group or a $C_3$- or $C_4$-dihydroxyalkyl group.

10. 2-hydroxy-3-mercaptopropionic acid (2'-hydroxyethyl) ester.

11. 2-hydroxy-3-mercaptopropionic acid (2',3'-dihydroxypropyl) ester.

## Revendications

1. Agent pour la mise en pli permanente des cheveux à base d'une matière réductrice de la kératine, caractérisé en ce que comme matière réductrice de la kératine, il contient un ester de l'acide 2-hydroxy-3-mercaptopropionique de formule (I)

$$HS\text{-}CH_2\text{-}CH(OH)\text{-}COOR \quad (I)$$

R étant un groupe alkyle, un groupe hydroxyalkyle ou un groupe dihydroxyalkyle.

2. Agent sel on la revendication 1 caractérisé en ce que le reste R de formule (I) est un groupe alkyle en $C_1$ à $C_4$, un groupe hydroxyalkyle en $C_2$ à $C_4$, ou un groupe dihydroxyalkyle en $C_3$ à $C_4$.

3. Agent selon la revendication 1 ou 2, caractérisé en ce qu'il contient comme seule matière réductrice de la kératine un ester de l'acide 2-hydroxy-3-mercaptopropionique.

4. Agent sel on l'une des revendications 1 à 3, caractérisé en ce que l'ester d'acide 2-hydroxy-3-mercaptopropionique de formule (I) est l'ester éthylique de l'acide 2-hydroxy-3-mercaptopropionique, le 2'-(hydroxyéthyl)ester de l'acide 2-hydroxy-3-mercaptopropionique, ou le (2',3'-dihydroxypropyl)ester de l'acide 2-hydroxy-3-mercaptopropionique.

5. Agent selon l'une des revendications 1 à 4, caractérisé en ce que l'ester de l'acide 2-hydroxy-3-mercaptopropionique de formule (I) dans l'agent prêt à l'emploi est contenu à raison de 4 à 30% en poids.

**6.** Agent selon l'une des revendications 1 à 5, caractérisé en ce que la valeur de pH de l'agent prêt à l'emploi va de 4,5 à 9.

**7.** Agent sel on l'une des revendications 1 à 6, caractérisé en ce qu'il est préparé avant emploi par mélange de deux ou trois composants, le composant contenant l'ester de l'acide 2-hydroxy-3-mercaptopropionique de formule (I) est à l'état anhydre.

**8.** Ester de l'acide 2-hydroxy-3-mercaptopropionique de formule (II)

$$HS\text{-}CH_2\text{-}CH(OH)\text{-}COOR^1 \qquad (II)$$

avec $R^1$ = un groupe alkyle avec au moins 3 atomes de carbone, un groupe hydroxyalkyle ou un groupe dihydroxyalkyle.

**9.** Composé selon la revendication 8, caractérisé en ce que dans la formule (II), le reste $R^1$ est un groupe alkyle en $C_3$ ou $C_4$, un groupe hydroxyalkyle en $C_2$ à $C_4$ ou un groupe dihydroxyalkyle en $C_3$ ou $C_4$.

**10.** (2'-hydroxyéthyl)ester de l'acide 2-hydroxy-3-mercaptopropionique.

**11.** (2',3'-dihydroxypropyl)ester de l'acide 2-hydroxy-3-mercaptopropionique.